# EUROPÄISCHE PATENTSCHRIFT

(11) **EP 3 995 119 B1**
(45) Veröffentlichungstag und Bekanntmachung des Hinweises auf die Patenterteilung: **03.04.2024**
(21) Anmeldenummer: 21205253.4
(22) Anmeldetag: 28.10.2021
(51) Int. Cl.: A61F 13/15, A61F 13/511, A61F 13/537

(54) **VERFAHREN ZUM BEHANDELN EINES VLIESMATERIALS**
METHOD FOR TREATING A NON-WOVEN MATERIAL
PROCÉDÉ DE TRAITEMENT D'UNE MATIÈRE NON-TISSÉE

(30) Priorität: 05.11.2020 DE 102020129203
(43) Veröffentlichungstag der Anmeldung: 11.05.2022
(73) Patentinhaber: Paul Hartmann AG, 89522 Heidenheim (DE)
(72) Erfinder: Bährle, Christian, 89542 Herbrechtingen (DE); Krämer, Tobias, 91443 Scheinfeld (DE)
(74) Vertreter: DREISS Patentanwälte PartG mbB

(56) Entgegenhaltungen:
- WO-A1-2020/131663
- US-A1- 2017 258 649
- US-A1- 2019 328 588

## Beschreibung

Die Erfindung betrifft ein Verfahren zum Behandeln eines Vliesmaterials. Endlos zuführbare, also bahnförmige Vliesmaterialien, finden bei der Herstellung von absorbierenden Hygieneartikeln für den einmaligen Gebrauch in großem Umfang Verwendung. Sie werden typischerweise in Form von Rollen als Lagerform vorgehalten und von diesen Rollen als endloses bahnförmiges Vliesmaterial dem Herstellungsprozess zugeführt. Die hier in Rede stehenden Vliesmaterialien finden Verwendung zur Herstellung von Decklagen (Topsheets), Rückenlagen (Backsheets), Absorptionskörperschichten, hinten oder vorn an Hygieneartikel angefügten Seitenabschnitten oder Verschlussmittelkomponenten, seitlichen Barriereelementen oder dergleichen Komponenten, sowie als Flüssigkeitsaufnahme- und -Verteilerschichten.

Wenn bahnförmige Vliesmaterialen als Rollenware aufgerollt und vorgehalten werden, so führt dies oftmals zu einer Verdichtung des bahnförmigen Vliesmaterials, was auf den Vorgang des Aufrollens unter Zugspannung der Vliesmaterialbahn zurückzuführen ist. Das wieder abgerollte Vliesmaterial wird dann mitunter als zu stark komprimiert angesehen, weil es eine als zu gering empfundene Dicke aufweist, oder es wird haptisch als zu stark komprimiert empfunden, weil es mitunter auch eine wenig textile Anmutung vermittelt. Bislang wurde dies hingenommen, oder man vertraute darauf, dass sich im Zuge der Handhabung eines bahnförmigen zugeführten Vliesmaterials in der Herstellungsmaschine ein gewisses Aufbauschen des Vliesmaterials wieder einstellt. Es wurde aber auch bereits vorgeschlagen, durch Zuführung von Wärme, insbesondere in Form von Infrarotstrahlung, verdichtete bahnförmige Vliesmaterialien wieder aufzubauschen. Mit WO 2020/131663 A1 wurde der Vorschlag unterbreitet, das aufzubauschende Vliesmaterial mittels einer rasch stellbaren Tänzeranordnung einer Infrarotstrahlungsquelle zuzuführen und wieder zu entfernen, damit bei plötzlichem Maschinenstillstand die Trägheit der Infrarotstrahlungsquelle nicht zu Materialschäden bei dem Vliesmaterial führt und das Verfahren nach Unterbrechung auch rasch wieder angefahren werden kann.

US 2019/0328588 A1 offenbart ein Verfahren zum Behandeln eines Vliesmaterials zur Erzeugung einer 3-dimensionalen Struktur, bei dem die Schritte
- Bereitstellen eines bahnförmigen Vliesmaterials
- Fördern des Vliesmaterials
- Zuführen des Vliesmaterials
- Dehnen des Vliesmaterials
ausgeführt werden. Vor dem Verfahrensschritt "Dehnen" werden in dem insbesondere endlos zugeführten bahnförmigen Vliesmaterial in Maschinenrichtung erstreckte Schlitze oder Schwächungslinien ausgebildet werden, so dass durch das Dehnen des bahnförmigen Vliesmaterials Öffnungen und damit die 3-dimensionalen Struktur entstehen.

Der vorliegenden Erfindung liegt die Aufgabe zugrunde, ein Verfahren zum Behandeln eines Vliesmaterials anzugeben, mit dem die eingangs geschilderten Probleme auf betriebssichere und wirtschaftlich ausführbare Weise gelöst werden können. Hierfür wird ein Verfahren angegeben zum Behandeln, und zwar zum Aufbauschen eines Vliesmaterials, umfassend die folgenden Schritte:
- Bereitstellen eines bahnförmigen Vliesmaterials mit einer Bahnebene, einer Längsrichtung und einer Querrichtung, wobei das Vliesmaterial in der Querrichtung eine erste effektive Quererstreckung Q1, eine erste Dicke D1 und ein erstes Flächengewicht FG1, jeweils innerhalb der ersten effektiven Quererstreckung Q1, aufweist,
- Fördern des Vliesmaterials in einer Maschinenrichtung, die der Längsrichtung des bahnförmigen Vliesmaterials entspricht,
- wobei das Vliesmaterial ein in der Längsrichtung und Maschinenrichtung kardiertes und heißluftverfestigtes Vliesmaterial ist, welches in der Folge der Heißluftverfestigung eine Vielzahl von Schmelzbindungen zwischen Fasern des Vliesmaterials aufweist,
- Zuführen des Vliesmaterials zu einer Dehnungsstation,
- Dehnen des Vliesmaterials in der Dehnungsstation in der Querrichtung, orthogonal zur Maschinenrichtung, und in der Bahnebene, derart dass zumindest ein Teil der Schmelzbindungen aufgebrochen und gelöst wird,
- wobei bei dem Vliesmaterial infolge des Dehnens und nach Wegnahme einer die Dehnung bewirkenden Maßnahme eine zweite effektive Quererstreckung Q2, und eine zweite Dicke D2 und ein zweites Flächengewicht FG2, jeweils innerhalb der zweiten effektiven Quererstreckung Q2, gebildet wird,
- wobei die zweite effektive Quererstreckung Q2 größer ist als die erste effektive Quererstreckung Q1,
- wobei das zweite Flächengewicht FG2 geringer ist als das erste Flächengewicht FG1,
wobei die zweite Dicke D2 größer ist als die erste Dicke D1.

Im Zuge der Handhabung von Vliesmaterialien wurde bei der Anmelderin auf überraschende Weise festgestellt, dass eine Querdehnung von gewissen mit einer Vorzugsrichtung ausgebildeten Vliesmaterialien zu einer aufbauschenden Wirkung führen bzw. zur Erzielung einer aufbauschenden Wirkung genutzt werden kann.

Es wurde erfindungsgemäß erkannt, dass ein kardiertes und heißluftverfestigtes Vliesmaterial mit einer Vielzahl von Schmelzbindungen zwischen Fasern des Vliesmaterials bei einer Dehnung quer zur Kardierungsrichtung, vorliegend quer zur Maschinenrichtung, zu einem Aufbauschen des Vliesmaterials führt. Beim Dehnen des Vliesmaterials wird nämlich zumindest ein Teil der Schmelzbindungen zwischen Fasern des Vliesmaterials aufgebrochen, und es wird diesseits davon ausgegangen, dass das Vliesmaterial hierdurch Freiheitsgrade bei den einzelnen Fasern zurückerhält, so dass die durch die vorherige thermische Verfestigung eingebrachte und fixierte Eigenspannung der Fasern freigesetzt werden kann und zu einer dementsprechenden Entspannung des dreidimensionalen Faserverbunds oder Fasernetzwerks führen kann, die wiederum zu dem dreidimensional aufbauschenden Effekt bei der Vliesstruktur führt.

Es war hierbei weiter überraschend, dass typische Performanceparameter, wie die Flüssigkeitsdurchtrittszeit und das Rücknässungsverhalten, bei auf erfindungsgemäße Weise behandelten Vliesmaterialien der hier in Rede stehenden beanspruchten Art hierdurch nicht oder zumindest nicht wesentlich verschlechtert wurden, so dass die auf erfindungsgemäße Weise behandelten Vliesmaterialien für den intendierten Einsatz nicht weniger, sondern mitunter sogar besser geeignet waren. Insbesondere hat sich die Verwendbarkeit eines erfindungsgemäß behandelten Vliesmaterials als Flüssigkeitsaufnahme- und - verteilerschicht bei absorbierenden Hygieneartikeln durch das Aufbauschen sogar verbessert.

Wenn in dieser Anmeldung von einer ersten effektiven Quererstreckung Q1 und von einer zweiten effektiven Quererstreckung Q2 die Rede ist, so ist hiermit eine Quererstreckung des bahnförmigen Vliesmaterials gemeint, welche sich beim Einspannen in beidseits vorgesehene Klemmen oder Nadelwalzen oder andere in der Dehnungsstation an der Vliesmaterialbahn seitlich angreifende Stellelemente in Querrichtung zwischen diesen Klemmen oder Nadelwalzen oder Stellelementen erstreckt. Es bezeichnet also diejenige Quererstreckung des Vliesmaterials, die außerhalb des Angriffsbereichs von Klemmen, Nadelwalzen oder anderen Stellelementen, einer Dehnung unterworfen wird. Diejenige Quererstreckung des Vliesmaterials, die durch seitliche Klemmen, Nadelwalzen oder sonstige Stellelementen unmittelbar beaufschlagt wird, wird nicht zu der effektiven ersten Quererstreckung Q1 vor dem Dehnen bzw. zu der zweiten effektiven Quererstreckung Q2 nach dem Dehnen hinzugerechnet. Sofern das Dehnen in der Querrichtung nicht durch Angriff seitlicher Klemmen, Nadelwalzen oder sonstiger Stellelemente sondern in anderer Weise erfolgt, also beispielsweise durch Zuführen des bahnförmigen Vliesmaterials zu einer Ringroll-Einrichtung (englisch "ringrolling"), kann die erste effektive Quererstreckung Q1 und auch die zweite effektive Quererstreckung Q2 die gesamte Breite oder Erstreckung des bahnförmigen Vliesmaterials in der Querrichtung vor dem Dehnen bzw. nach dem Dehnen bedeuten.

Es ist wesentlich, dass beim Dehnen des Vliesmaterials in der Querrichtung Schmelzbindungen zwischen Fasern des Vliesmaterials aufgebrochen und gelöst werden, damit die im heißluftverfestigten Vliesmaterial gespeicherte Spannungsenergie zumindest teilweise freigesetzt und zur Erzielung des aufbauschenden Effekts des Vliesmaterials umgesetzt werden kann.

Da nach dem Dehnen auch wieder elastisch wirkende Rückstellkräfte innerhalb des Vliesmaterials zur Wirkung gelangen können, nachdem das Vliesmaterial in der Querrichtung im Wesentlichen kraftfrei gestellt wurde, d.h. auf das Vliesmaterial einwirkende dehnende Kräfte in der Querrichtung weggenommen wurden, kann sich die beim Dehnen aufgezwungene Quererstreckung des Vliesmaterials teilweise wieder zurückbilden. Das Vliesmaterial wird aber recht zügig eine schlussendliche Quererstreckung, gegebenenfalls nach einer gewissen Dauer eines Entspannenlassens, einnehmen. Wenn das Vliesmaterial nach dem Verlassen der Dehnungsstation weiter unter einer auch recht geringen Zugspannung in der Maschinenrichtung steht, wird sich die schlussendliche Quererstreckung sehr rasch einstellen. In diesem dann stabilen Zustand wird die zweite effektive Quererstreckung Q2 des quer gedehnten Vliesmaterials und dessen zweite Dicke D2 und dessen zweites Flächengewicht FG2 ermittelt oder berechnet. Die Bestimmung des Flächengewichts FG1, FG2 erfolgt, indem die Masse eines typischerweise aus dem Flächenmaterial ausgestanzten Prüflings bestimmt und durch dessen Fläche geteilt wird. Das Flächengewicht kann gemäß Prüfverfahren nach DIN EN 29073-1:1992 ermittelt werden, wobei in Abweichung von dieser Norm die Größe der Prüflinge statt mit 500 cm², insbesondere mit 100 cm² oder mit 50 cm² gewählt werden können.

Bei der Ermittlung der ersten und der zweiten Dicke D1, D2 wird ein Prüfling des Vliesmaterials innerhalb der ersten bzw. der zweiten Quererstreckung Q1, Q2 gewonnen und unter einen Prüfdruck von 2 g/cm² gesetzt, und es wird dann insbesondere nach etwa 10 Sekunden unter diesem Prüfdruck die Dicke abgelesen. Die Bestimmung der Dicke kann insbesondere nach einem Prüfverfahren gemäß DIN EN ISO 9073-2:1997-02 ausgeführt werden.

Nach einer bevorzugten Ausführungsform des erfindungsgemäßen Verfahrens erweist es sich als vorteilhaft, wenn ein Verhältnis der zweiten effektiven Quererstreckung Q2 zur ersten effektiven Quererstreckung Q1 wenigstens 1,05, insbesondere wenigstens 1,10, insbesondere von wenigstens 1,15, insbesondere wenigstens 1,20 und insbesondere höchstens 1,70, insbesondere höchstens 1,60, insbesondere höchstens 1,50, insbesondere höchstens 1,40 beträgt.

Es erweist sich als vorteilhaft, wenn das erfindungsgemäße Verfahren unter Verwendung eines Vliesmaterials ausgeführt wird, bei dem das erste Flächengewicht FG1 des Vliesmaterials einen Wert von wenigstens 20 g/m², insbesondere von wenigstens 25 g/m², insbesondere von wenigstens 30 g/m², insbesondere von höchstens 60 g/m², insbesondere von höchstens 50 g/m², insbesondere von höchstens 45 g/m², aufweist.

Weiter erweist es sich als vorteilhaft, wenn ein Verhältnis des ersten Flächengewichts FG1 zum zweiten Flächengewicht FG2 einen Wert von wenigstens 1,05, insbesondere von wenigstens 1,10, insbesondere von wenigstens 1,20 und insbesondere höchstens 1,70, insbesondere höchstens 1,60, insbesondere höchstens 1,50, insbesondere höchstens 1,40 beträgt.

Bei hier in Rede stehenden kardierten und heißluftverfestigten Vliesmaterialien erweist es sich als vorteilhaft, wenn das Dehnen in der Dehnungsstation entlang der Querrichtung um wenigstens 10 %, insbesondere um wenigstens 20 %, insbesondere um wenigstens 30 %, und um höchstens 100 %, insbesondere um höchstens 80 %, insbesondere um höchstens 60 %, und vorzugsweise um 40-60 % der effektiven Quererstreckung Q1 erfolgt. Hierbei wird ein wesentlicher Anteil der Schmelzbindungen zwischen Fasern des Vliesmaterials aufgebrochen, so dass sich die aufbauschende Wirkung in einem für praktische Anwendungen geeigneten Maß einstellt.

Es erweist sich weiter als vorteilhaft, wenn ein Verhältnis der ersten Dicke D1 zu der zweiten Dicke D2 wenigstens 0,30, insbesondere wenigstens 0,40, insbesondere wenigstens 0,50, und höchstens 0,80, insbesondere höchstens 0,75, insbesondere höchstens 0,70 beträgt.

Weiter erweist es sich als vorteilhaft, wenn das Vliesmaterial flüssigkeitsdurchlässig ausgebildet ist, so dass es als Topsheet oder Flüssigkeitsaufnahme- oder - verteilerschicht bei einem absorbierenden Hygieneartikel verwendet werden kann.

Als besonders vorteilhaft erweist es sich, wenn das Vliesmaterial Mehrkomponenten-Fasern, insbesondere Bikomponenten-Fasern, insbesondere Bikomponenten-Polyesterfasern, umfasst oder daraus besteht. Bei diesen Mehrkomponenten-Fasern, insbesondere Bikomponentenfasern, kann es sich um sogenannte "Kern/Mantel-Fasern" oder um Seite-an-Seite-Fasern handeln. Bei solchen Fasern wird durch die Ausbildung von Schmelzbindungen und deren erfindungsgemäßes Aufbrechen durch Dehnen in der Querrichtung in besonders hohem Maße verspannende Energie gespeichert und wieder freigesetzt werden.

Im Hinblick auf die weitere Handhabung des Vliesmaterials kann es sich als vorteilhaft erweisen, wenn das Vliesmaterial einer der Dehnungsstation nachgeordneten Trennstation zugeführt wird, wobei es durch Trennen in der Querrichtung vereinzelt wird. Solchenfalls können vereinzelte Abschnitte des Vliesmaterials in eine Lagerform überführt oder unmittelbar weiterverarbeitet, insbesondere der Herstellung eines absorbierenden Hygieneartikels zugeführt werden.

Es ist aber auch denkbar und für bestimmte Anwendungen vorteilhaft, wenn das Vliesmaterial nach dem Verfahrensschritt des Dehnens und gegebenenfalls des Entspannenlassens in eine Lagerform überführt wird. Es kann insbesondere nach dem Verfahrensschritt des Dehnens und gegebenenfalls des Entspannenlassens in die Lagerform aufgerollt werden. Das Vliesmaterial kann so zu einem späteren Zeitpunkt einer an sich beliebigen Verwendung, insbesondere einem Herstellungsprozess für Hygieneartikel, zugeführt werden.

Ebenso ist es denkbar, dass der eingangs erwähnte Verfahrensschritt des Bereitstellens des bahnförmigen Vliesmaterials ein Abrollen des Vliesmaterials von einer Rolle umfasst.

Gegenstand der vorliegenden Erfindung ist aber auch ein Verfahren zum Aufbauschen eines Vliesmaterials, bei dem das quergedehnte Vliesmaterial für die weitere Verwendung bei der Herstellung von absorbierenden Hygieneartikeln "in-line", also ohne zwischenzeitliches Überführen in eine Lagerform, einem Herstellungsprozess von absorbierenden Hygieneartikeln zugeführt wird und das noch bahnförmige oder bereits vereinzelte Vliesmaterial an Komponenten des herzustellenden Hygieneartikels, insbesondere als körperzugewandte Deckschicht oder als Flüssigkeitsaufnahme- oder -Verteilerschicht, angefügt wird. Hierbei ist der Begriff des Anfügens im weitesten Sinne zu verstehen und erfasst stoffschlüssiges Fügen oder Fügen mittels weiterer Fügeelemente oder auch nur eine bestimmungsgemäße dauerhafte Anordnung des erfindungsgemäß behandelten Vliesmaterials in dem Hygieneartikel.

Gegenstand der Erfindung ist auch ein Verfahren zum Herstellen von absorbierenden Hygieneartikeln, umfassend ein Verfahren zum Behandeln eines Vliesmaterials nach Anspruch 14.

Weitere Einzelheiten, Merkmale und Vorteile der Erfindung ergeben sich aus den beigefügten Patentansprüchen und aus der zeichnerischen Darstellung und nachfolgenden Beschreibung einer bevorzugten Ausführungsform der Erfindung.

In der Zeichnung zeigt:
- Figuren 1a, 1b: eine stark schematisierte Längsschnittansicht und eine Draufsicht auf schematisch angedeutete Stationen bei der Ausführung des erfindungsgemäßen Verfahrens zum Behandeln eines Vliesmaterials;
- Figuren 2a, 2b: den Figuren 1a, 1b entsprechende Ansichten, wobei das Vliesmaterial vereinzelt wird; und
- Figuren 3a, 3b: den Figuren 1a, 1b entsprechende Darstellungen, wobei das Vliesmaterial einem Herstellungsprozess absorbierender Hygieneartikel zugeführt wird.

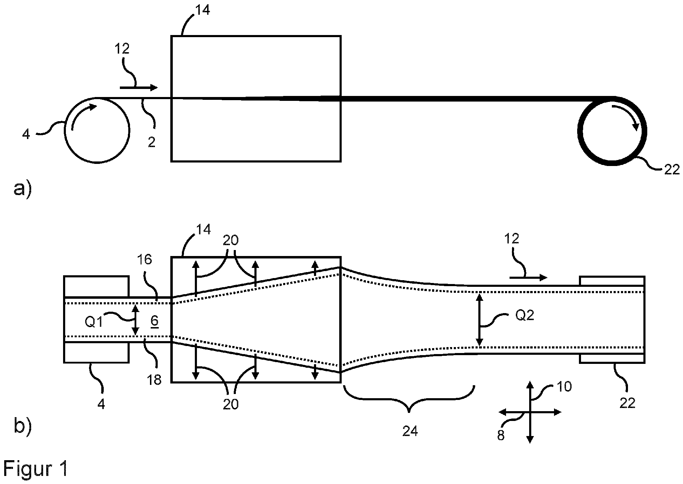

Die Figuren 1a, 1b zeigen in stark schematisierter Form und Darstellung ein erfindungsgemäßes Verfahren zum Aufbauschen eines Vliesmaterials. Das Vliesmaterial wird als auf eine Rolle oder Haspel als Lagerform aufgerolltes bahnförmiges Vliesmaterial 2 bereitgestellt. Das Vliesmaterial 2 umfasst eine Bahnebene 6, eine Längsrichtung 8 und eine Querrichtung 10.

Bei dem Vliesmaterial 2 handelt es sich um ein in der Längsrichtung 8 kardiertes und heißluftverfestigtes Vliesmaterial der eingangs beschriebenen Art, welches in der Folge der Heißluftverfestigung eine Vielzahl von Schmelzbindungen zwischen Fasern des Vliesmaterials 2 aufweist.

Das bahnförmige Vliesmaterial 2 wird von der Rolle oder Haspel 4 in einer Maschinenrichtung 12, die parallel zur Längsrichtung 8 des bahnförmigen Vliesmaterials 2 verläuft, abgerollt und einer Dehnungsstation 14 zugeführt. Das Vliesmaterial 2 wird in der Dehnungsstation 14 in seiner Bahnebene 6 und in der Querrichtung 10 gedehnt. Die Dehnung erfolgt - wie eingangs dargelegt - derart, dass Schmelzbindungen zwischen Fasern des kardierten und heißluftverfestigten Vliesmaterials 2 aufgebrochen und gelöst werden. Dieser Vorgang des Dehnens des Vliesmaterials 2 ist in der Figur 1b rein schematisch dargestellt. Das Dehnen kann auf verschiedene Weise erfolgen. Letzten Endes müssen auf das Vliesmaterial Kräfte eingeleitet werden, die diese Dehnung herbeiführen. Beispielsweise wäre es denkbar - wie in der Figur 1b angedeutet -, dass das Vliesmaterial 2 an seinen beiden in der Querrichtung 10 gegenüberliegenden Längsrandbereichen 16, 18 in geeigneter Form ergriffen und dann in Richtung der in Figur 1b angedeuteten Pfeile 20 dehnend auseinandergezogen wird. Dieses Ergreifen der Längsrandbereiche 16, 18, die in der Figur 1b nach innen durch eine gepunktete Linie angedeutet und begrenzt sind, kann beispielsweise durch Klemmen oder durch Eingriff von schräggestellten Nadelwalzen oder in sonstiger Weise erfolgen. Der Umfang der Querdehnung des Vliesmaterials 2 kann wie eingangs erläutert gewählt werden. Wie eingangs ebenfalls erläutert, wurde erfindungsgemäß festgestellt, dass das Aufbrechen und Lösen von Schmelzbindungen zwischen Fasern des Vliesmaterials durch die Querdehnung zu einem Aufbauschen des Vliesmaterials orthogonal zur Bahnebene 6 führt, also zu einer Zunahme der Dicke des Vliesmaterials 2.

Wenn das bahnförmige Vliesmaterial wie dargestellt vorzugsweise kontinuierlich, jedoch nicht zwingend kontinuierlich gedehnt wurde und schließlich die Dehnungsstation 14 in der Maschinenrichtung 12 verlässt, so behält es die zuvor durch Dehnung aufgezwungene Quererstreckung nicht stets vollständig bei, sondern es kann sich je nach Material wieder teilweise, jedoch keineswegs vollständig zusammenziehen. Das Vliesmaterial 2 kann dann wie in Figur 1b beispielhaft angedeutet in eine Lagerform, insbesondere wiederum eine Rolle oder Haspel 22 aufgerollt werden.

Um den Effekt des Aufbauschens des Vliesmaterials 2 quantitativ erfassen und beschreiben zu können, wird - wie in Figur 1b angedeutet - eine erste effektive Quererstreckung Q1 des geförderten bahnförmigen Vliesmaterials bestimmt. Wie durch die gepunktete Linie angedeutet, wird die Quererstreckung des Vlieses im Bereich der Längsrandbereiche 16, 18, wo die die Dehnung des Vliesmaterials herbeiführenden Kräfte eingeleitet werden, bei der Bestimmung der ersten effektiven Quererstreckung Q1 vor der Dehnung und bei der Bestimmung der zweiten effektiven Quererstreckung Q2 nach der Dehnung nicht mitberücksichtigt. Die Quererstreckung Q2 nach der Dehnung wird erst bestimmt, wenn sich die Quererstreckung des Vliesmaterials nicht weiter ändert, sondern konstant bleibt. In der Figur 1b ist hierfür mit der geschweiften Klammer 24 ein Verfahrensintervall des Entspannenlassens des Vliesmaterials angedeutet. Gleichermaßen wird eine erste Dicke D1 und ein erstes Flächengewicht FG1 des Vliesmaterials 2 im Bereich der ersten effektiven Quererstreckung Q1 des zugeführten Vliesmaterials 2 im Ausgangszustand, also vor der Dehnungsstation 14, bestimmt. Ferner wird eine zweite Dicke D2 und ein zweites Flächengewicht FG2 des Vliesmaterials jeweils innerhalb der zweiten effektiven Quererstreckung Q2 in einem Zustand konstanter Quererstreckung des Vliesmaterials 2 nach der Dehnung bestimmt. Erfindungsgemäß wurde festgestellt, dass dabei die zweite Dicke D2 des Vliesmaterials 2 größer ist als die erste Dicke D1, das Vliesmaterial also durch eine Querdehnung trotz Vergrößerung der effektiven Quererstreckung eine größere Dicke aufweist als im Ausgangszustand, also gegenüber dem Ausgangszustand auf erfindungsgemäße Weise aufgebauscht wurde.

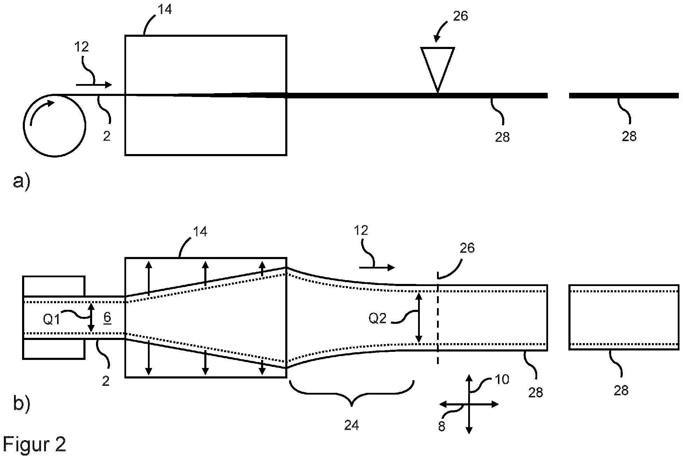

Die Figuren 2a, 2b verdeutlichen eine Verfahrensführung, bei der das quergedehnte Vliesmaterial 2 nicht wieder in eine Lagerform aufgerollt wird, sondern nach dem Verfahrensschritt des Querdehnens in der Dehnungsstation 14 einer schematisch angedeuteten Trennstation 26 zugeführt wird, wo es orthogonal zur Maschinenrichtung 12 in einzelne Vliesmaterialabschnitte 28 vereinzelt wird. Diese Vereinzelung kann auch schon vorgenommen werden, bevor das quergedehnte Vliesmaterial auf seine schlussendliche Quererstreckung relaxiert ist. Bevorzugt erfolgt jedoch die Ausführung des Vereinzelns erst, wenn das Vliesmaterial seine stabile Quererstreckung erreicht hat.

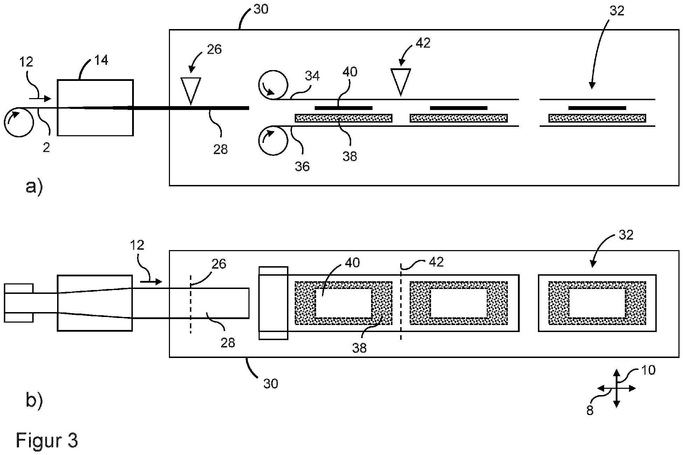

Schließlich verdeutlichen die Figuren 3a, 3b ein Verfahren, bei dem das quergedehnte Vliesmaterial für die weitere Verwendung bei der Herstellung von absorbierenden Hygieneartikeln "in-line", also ohne zwischenzeitliches Überführen in eine Lagerform, einem Herstellungsprozess von absorbierenden Hygieneartikeln zugeführt wird, und bei dem das noch bahnförmige oder bereits vereinzelte Vliesmaterial an Komponenten des herzustellenden Hygieneartikels, insbesondere als körperzugewandte Deckschicht oder als Flüssigkeitsaufnahme- oder -Verteilerschicht, angefügt wird.

Dieser Herstellungsprozess ist in Figuren 3a, 3b mit dem Bezugszeichen 30 bezeichnet, wobei beispielhaft das quergedehnte Vliesmaterial als endlose Bahn diesem Herstellungsprozess 30 zugeführt und erst dort mittels einer Trenneinrichtung 26 vereinzelt wird. Auch die Zuführung von Komponenten der herzustellenden Hygieneartikel 32 in Form einer Deckschicht 34, einer Rückenschicht 36, eines Absorptionskörpers 38 ist rein schematisch und weder abschließend noch maßstabsgetreu dargestellt. In diesem schematisch dargestellten Fall werden durch die Trennstation 26 vereinzelte Vliesmaterialabschnitte 28 als Flüssigkeitsaufnahme- und - verteilerschicht 40 in dem herzustellenden Hygieneartikel 32 verwendet bzw. angeordnet. Eine weitere Trenneinrichtung 42 ist innerhalb des Herstellungsprozesses 30 zur Vereinzelung der herzustellenden Hygieneartikel 32 angedeutet.

## Patentansprüche

1. Verfahren zum Behandeln eines Vliesmaterials (2), nämlich zum Aufbauschen eines Vliesmaterials (2), umfassend folgende Schritte:
- Bereitstellen eines bahnförmigen Vliesmaterials (2) mit einer Bahnebene (6), einer Längsrichtung (8) und einer Querrichtung (10), wobei das Vliesmaterial (2) in der Querrichtung (10) eine erste effektive Quererstreckung Q1, eine erste Dicke D1 und ein erstes Flächengewicht FG1, jeweils innerhalb der ersten effektiven Quererstreckung Q1, aufweist,
- Fördern des Vliesmaterials (2) in einer Maschinenrichtung (12), die der Längsrichtung (8) des bahnförmigen Vliesmaterials (2) entspricht,
- wobei das Vliesmaterial (2) ein in der Längsrichtung (8) und Maschinenrichtung (12) kardiertes und heißluftverfestigtes Vliesmaterial (2) ist, welches in der Folge der Heißluftverfestigung eine Vielzahl von Schmelzbindungen zwischen Fasern des Vliesmaterials (2) aufweist,
- Zuführen des Vliesmaterials (2) zu einer Dehnungsstation (14),
- Dehnen des Vliesmaterials (2) in der Dehnungsstation (14) in der Querrichtung (10), orthogonal zur Maschinenrichtung (12), und in der Bahnebene (6) , derart dass zumindest ein Teil der Schmelzbindungen aufgebrochen und gelöst wird,
- wobei bei dem Vliesmaterial (2) infolge des Dehnens und nach Wegnahme einer die Dehnung bewirkenden Maßnahme eine zweite effektive Quererstreckung Q2, und eine zweite Dicke D2 und ein zweites Flächengewicht FG2, jeweils innerhalb der zweiten effektiven Quererstreckung Q2, gebildet wird,
- wobei die zweite effektive Quererstreckung Q2 größer ist als die erste effektive Quererstreckung Q1,
- wobei das zweite Flächengewicht FG2 geringer ist als das erste Flächengewicht FG1,
wobei die zweite Dicke D2 größer ist als die erste Dicke D1.

2. Verfahren nach Anspruch 1, **dadurch gekennzeichnet, dass** ein Verhältnis der zweiten effektiven Quererstreckung Q2 zur ersten effektiven Quererstreckung Q1 wenigstens 1,05, insbesondere wenigstens 1,10, insbesondere von wenigstens 1,15, insbesondere wenigstens 1,20 und insbesondere höchstens 1,70, insbesondere höchstens 1,60, insbesondere höchstens 1,50, insbesondere höchstens 1,40 beträgt.

3. Verfahren nach Anspruch 1 oder 2, **dadurch gekennzeichnet, dass** das erste Flächengewicht FG1 des Vliesmaterials (2) einen Wert von wenigstens 20 g/m², insbesondere von wenigstens 25 g/m², insbesondere von wenigstens 30 g/m², insbesondere von höchstens 60 g/m², insbesondere von höchstens 50 g/m², insbesondere von höchstens 45 g/m², aufweist.

4. Verfahren nach einem oder mehreren der vorangegangenen Ansprüche, **dadurch gekennzeichnet, dass** ein Verhältnis des ersten Flächengewichts FG1 zum zweiten Flächengewicht FG2 einen Wert von wenigstens 1,05, insbesondere von wenigstens 1,10, insbesondere von wenigstens 1,20 und insbesondere höchstens 1,70, insbesondere höchstens 1,60, insbesondere höchstens 1,50, insbesondere höchstens 1,40 beträgt.

5. Verfahren nach einem oder mehreren der vorangegangenen Ansprüche, **dadurch gekennzeichnet, dass** das Dehnen in der Dehnungsstation (14) entlang der Querrichtung (10) um wenigstens 10 %, insbesondere um wenigstens 20 %, insbesondere um wenigstens 30 %, und um höchstens 100 %, insbesondere um höchstens 80 %, insbesondere um höchstens 60 %, und vorzugsweise um 40-60 % der effektiven Quererstreckung Q1 erfolgt.

6. Verfahren nach einem oder mehreren der vorangegangenen Ansprüche, **dadurch gekennzeichnet, dass** ein Verhältnis der ersten Dicke D1 zu der zweiten Dicke D2 wenigstens 0,30, insbesondere wenigstens 0,40, insbesondere wenigstens 0,50, und höchstens 0,80, insbesondere höchstens 0,75, insbesondere höchstens 0,70 beträgt.

7. Verfahren nach einem oder mehreren der vorangegangenen Ansprüche, **dadurch gekennzeichnet, dass** das Vliesmaterial (2) flüssigkeitsdurchlässig ist.

8. Verfahren nach einem oder mehreren der vorangegangenen Ansprüche, **dadurch gekennzeichnet, dass** das Vliesmaterial (2) Mehrkomponenten-Fasern, insbesondere Bikomponenten-Fasern, insbesondere Bikomponenten-Polyesterfasern, umfasst oder daraus besteht.

9. Verfahren nach einem oder mehreren der vorangegangenen Ansprüche, **dadurch gekennzeichnet, dass** das Vliesmaterial (2) einer der Dehnungsstation (14) nachgeordneten Trennstation (26) zugeführt wird, wobei es durch Trennen in der Querrichtung vereinzelt wird.

10. Verfahren nach einem oder mehreren der vorangegangenen Ansprüche, **dadurch gekennzeichnet, dass** das Vliesmaterial (2) nach dem Verfahrensschritt des Dehnens in eine Lagerform überführt wird.

11. Verfahren nach dem vorangegangenen Anspruch 10, **dadurch gekennzeichnet, dass** das Vliesmaterial (2) in die Lagerform aufgerollt wird.

12. Verfahren nach einem oder mehreren der vorangegangenen Ansprüche, **dadurch gekennzeichnet, dass** das Bereitstellen des bahnförmigen Vliesmaterials (2) ein Abrollen des Vliesmaterials von einer Rolle (4) umfasst.

13. Verfahren nach einem oder mehreren der vorangegangenen Ansprüche, **dadurch gekennzeichnet, dass** das Vliesmaterial (2) für die weitere Verwendung bei der Herstellung von absorbierenden Hygieneartikeln "in-line", also ohne zwischenzeitliches Überführen in eine Lagerform, einem Herstellungsprozess (30) von absorbierenden Hygieneartikeln (32) zugeführt wird und dass das noch bahnförmige oder bereits vereinzelte Vliesmaterial (2) an Komponenten des herzustellenden Hygieneartikels (32), insbesondere als körperzugewandte Deckschicht (34) oder als Flüssigkeitsaufnahme- oder -Verteilerschicht (40), angefügt wird.

14. Verfahren zum Herstellen von absorbierenden Hygieneartikeln (32), umfassend ein Verfahren zum Behandeln eines Vliesmaterials (2) nach Anspruch 13.

## Claims

1. Method for treating a nonwoven material (2), namely for bulking a nonwoven material (2), comprising the following steps:
- providing a web-like nonwoven material (2) having a web plane (6), a longitudinal direction (8) and a transverse direction (10), wherein the nonwoven material (2) has, in the transverse direction (10), a first effective transverse extension Q1, a first thickness D1, and a first weight per unit area FG1, in each case within the first effective transverse extension Q1,
- conveying the nonwoven material (2) in a machine direction (12) corresponding to the longitudinal direction (8) of the web-like nonwoven material (2),
- wherein the nonwoven material (2) is a thermobonded non-woven material (2) which is carded in the longitudinal direction (8) and machine direction (12) and which, as a result of the thermobonding, has a plurality of fusion bonds between fibers of the nonwoven material (2),
- feeding the nonwoven material (2) to a stretching station (14),
- stretching the nonwoven material (2) in the stretching station (14) in the transverse direction (10), orthogonal to the machine direction (12), and in the web plane (6), such that at least some of the fusion bonds are broken and detached,
- wherein a second effective transverse extension Q2, and a second thickness D2 and a second weight per unit area FG2, in each case within the second effective transverse extension Q2, are formed in the nonwoven material (2) as a result of the stretching and after removal of a measure effecting the stretching,
- wherein the second effective transverse extension Q2 is greater than the first effective transverse extension Q1,
- wherein the second weight per unit area FG2 is smaller than the first weight per unit area FG1,
wherein the second thickness D2 is greater than the first thickness D1.

2. Method according to claim 1, **characterized in that** a ratio of the second effective transverse extension Q2 to the first effective transverse extension Q1 is at least 1.05, in particular at least 1.10, in particular at least 1.15, in particular at least 1.20, and in particular at most 1.70, in particular at most 1.60, in particular at most 1.50, in particular at most 1.40.

3. Method according to claim 1 or 2, **characterized in that** the first weight per unit area FG1 of the nonwoven material (2) has a value of at least 20 g/m², in particular of at least 25 g/m², in particular of at least 30 g/m², in particular of at most 60 g/m², in particular of at most 50 g/m², in particular of at most 45 g/m ².

4. Method according to one or more of the preceding claims, **characterized in that** a ratio of the first weight per unit area FG1 to the second weight per unit area FG2 is a value of at least 1.05, in particular of at least 1.10, in particular of at least 1.20, and in particular at most 1.70, in particular at most 1.60, in particular at most 1.50, in particular at most 1.40.

5. Method according to one or more of the preceding claims, **characterized in that** the stretching in the stretching station (14) takes place in the transverse direction (10) by at least 10%, in particular by at least 20%, in particular by at least 30%, and by at most 100%, in particular by at most 80%, in particular by at most 60%, and preferably by 40-60% of the effective transverse extension Q1.

6. Method according to one or more of the preceding claims, **characterized in that** a ratio of the first thickness D1 to the second thickness D2 is at least 0.30, in particular at least 0.40, in particular at least 0.50, and in particular at most 0.80, in particular at most 0.75, in particular at most 0.70.

7. Method according to one or more of the preceding claims, **characterized in that** the nonwoven material (2) is liquid-permeable.

8. Method according to one or more of the preceding claims, **characterized in that** the nonwoven material (2) comprises or consists of multi-component fibers, in particular bicomponent fibers, in particular bicomponent polyester fibers.

9. Method according to one or more of the preceding claims, **characterized in that** the nonwoven material (2) is supplied to a separating station (26) downstream of the stretching station (14), wherein it is singularized by separation in the transverse direction.

10. Method according to one or more of the preceding claims, **characterized in that** the nonwoven material (2) is transferred into a storage shape after the stretching method step.

11. Method according to the preceding claim 10, **characterized in that** the nonwoven material (2) is rolled into the storage shape.

12. Method according to one or more of the preceding claims, **characterized in that** the provision of the web-like nonwoven material (2) comprises rolling the nonwoven material from a roller (4).

13. Method according to one or more of the preceding claims, **characterized in that** the nonwoven material (2), for further use in the production of absorbent sanitary articles, is supplied "in-line", i.e. without intermediate transfer into a storage shape, to a production process (30) for absorbent sanitary articles (32) and **in that** the still web-shaped or already singularized nonwoven material (2) is attached to components of the sanitary article (32) to be produced, in particular as a body-facing cover layer (34) or as a liquid absorption or distribution layer (40).

14. Method for producing absorbent sanitary articles (32), comprising a method for treating a nonwoven material (2) according to claim 13.

## Revendications

1. Procédé pour le traitement d'un matériau non tissé (2), à savoir pour redonner du gonflant à un matériau non tissé (2), comprenant les étapes suivantes:
- la fourniture d'un matériau non tissé (2) en forme de bande avec un plan de bande (6), une direction longitudinale (8) et une direction transversale (10), dans lequel le matériau non tissé (2) présente dans la direction transversale (10) une première étendue transversale effective Q1, une première épaisseur D1 et une première masse surfacique FG1, respectivement à l'intérieur de la première étendue transversale effective Q1,
- le transport du matériau non tissé (2) dans un sens machine (12), qui correspond à la direction longitudinale (8) du matériau non tissé (2) en forme de bande,
- dans lequel le matériau non tissé (2) est un matériau non tissé (2) cardé et renforcé à l'air chaud dans la direction longitudinale (8) et le sens machine (12), lequel présente à la suite du renforcement à l'air chaud une pluralité de liaisons par fusion entre les fibres du matériau non tissé (2),
- l'amenée du matériau non tissé (2) à un poste d'allongement (14),
- l'allongement du matériau non tissé (2) dans le poste d'allongement (14) dans la direction transversale (10), perpendiculairement au sens machine (12), et dans le plan de bande (6), de telle sorte qu'au moins une partie des liaisons par fusion est brisée et défaite,
- dans lequel pour le matériau non tissé (2) à la suite de l'allongement et après le retrait d'une mesure provoquant l'allongement une deuxième étendue transversale effective Q2, et une deuxième épaisseur D2 et une deuxième masse surfacique FG2, respectivement à l'intérieur de la deuxième étendue transversale effective Q2, sont formées
- dans lequel la deuxième étendue transversale effective Q2 est supérieure à la première étendue transversale effective Q1,
- dans lequel la deuxième masse surfacique FG2 est inférieure à la première masse surfacique FG1,
dans lequel la deuxième épaisseur D2 est supérieure à la première épaisseur D1.

2. Procédé selon la revendication 1, **caractérisé en ce qu'**un rapport de la deuxième étendue transversale effective Q2 sur la première étendue transversale effective Q1 atteint au moins 1,05, en particulier au moins 1,10, en particulier au moins 1,15, en particulier au moins 1,20 et en particulier au plus 1,70, en particulier au plus 1,60, en particulier au plus 1,50, en particulier au plus 1,40.

3. Procédé selon la revendication 1 ou 2, **caractérisé en ce que** la première masse surfacique FG1 du matériau non tissé (2) présente une valeur d'au moins 20 g/m², en particulier d'au moins 25 g/m², en particulier d'au moins 30 g/m², en particulier d'au plus 60 g/m², en particulier d'au plus 50 g/m², en particulier d'au plus 45 g/m².

4. Procédé selon l'une ou plusieurs des revendications précédentes, **caractérisé en ce qu'**un rapport de la première masse surfacique FG1 sur la deuxième masse surfacique FG2 atteint une valeur d'au moins 1,05, en particulier d'au moins 1,10, en particulier d'au moins 1,20 et en particulier d'au plus 1,70, en particulier d'au plus 1,60, en particulier d'au plus 1,50, en particulier d'au plus 1,40.

5. Procédé selon l'une ou plusieurs des revendications précédentes, **caractérisé en ce que** l'allongement dans le poste d'allongement (14) s'effectue le long de la direction transversale (10) d'au moins 10 %, en particulier d'au moins 20 %, en particulier d'au moins 30 %, et d'au plus 100 %, en particulier d'au plus 80 %, en particulier d'au plus 60 %, et de préférence de 40-60 % de l'étendue transversale effective Q1.

6. Procédé selon l'une ou plusieurs des revendications précédentes, **caractérisé en ce qu'**un rapport de la première épaisseur D1 sur la deuxième épaisseur D2 atteint au moins 0,30, en particulier au moins 0,40, en particulier au moins 0,50, et au plus 0,80, en particulier au plus 0,75, en particulier au plus 0,70.

7. Procédé selon l'une ou plusieurs des revendications précédentes, **caractérisé en ce que** le matériau non tissé (2) est perméable au liquide.

8. Procédé selon l'une ou plusieurs des revendications précédentes, **caractérisé en ce que** le matériau non tissé (2) comprend des fibres à plusieurs composants, en particulier des fibres à deux composants, en particulier des fibres polyester à deux composants ou en est constitué.

9. Procédé selon l'une ou plusieurs des revendications précédentes, **caractérisé en ce que** le matériau non tissé (2) est amené à un poste de séparation (26) situé en aval du poste d'allongement (14), dans lequel il est individualisé par séparation dans la direction transversale.

10. Procédé selon l'une ou plusieurs des revendications précédentes, **caractérisé en ce que** le matériau non tissé (2) après l'étape de procédé de l'allongement est transféré dans une forme de stockage.

11. Procédé selon la revendication précédente 10, **caractérisé en ce que** le matériau non tissé (2) est enroulé dans la forme de stockage.

12. Procédé selon l'une ou plusieurs des revendications précédentes, **caractérisé en ce que** la fourniture du matériau non tissé (2) en forme de bande comprend un déroulement du matériau non tissé d'un rouleau (4).

13. Procédé selon l'une ou plusieurs des revendications précédentes, **caractérisé en ce que** le matériau non tissé (2) pour l'utilisation ultérieure lors de la fabrication d'articles hygiéniques absorbants est amené «en ligne», par conséquent sans transfert intermédiaire dans une forme de stockage, à un processus de fabrication (30) d'articles hygiéniques absorbants (32) et que le matériau non tissé (2) encore en forme de bande ou déjà individualisé est joint aux composants de l'article hygiénique (32) à fabriquer, en particulier en tant que couche de recouvrement (34) tournée vers le corps ou en tant que couche d'absorption ou de répartition de liquide (40).

14. Procédé pour la fabrication d'articles hygiéniques absorbants (32), comprenant un procédé pour le traitement d'un matériau non tissé (2) selon la revendication 13.
